# EUROPEAN PATENT SPECIFICATION

(11) **EP 1 663 800 B1**
(45) Date of publication and mention of the grant of the patent: **09.06.2010**
(21) Application number: 04784600.1
(22) Date of filing: 21.09.2004
(51) Int. Cl.: A61M 1/02

(54) **PRESS FOR REMOVING SUPERNATANT FROM A FLEXIBLE VESSEL**
Presse zur Entfernung von Überstand aus einem flexiblen Behälter
PRESSE DESTINEE A ENLEVER UN SURNAGEANT D'UN CONTENANT SOUPLE

(30) Priority: 22.09.2003 US 504854 P
(43) Date of publication of application: 07.06.2006
(73) Proprietor: BATTELLE MEMORIAL INSTITUTE, Columbus, OH 43201-2693 (US)
(72) Inventor: MANK, James, F., Dublin, OH 43016 (US); BRESLER, Herbert, S., Bexley, OH 43209 (US)
(74) Representative: Müller-Boré & Partner Patentanwälte
(86) International application number: PCT/US2004/030784
(87) International publication number: WO 2005/030289

(56) References cited:
- EP-A1- 0 608 882
- WO-A-93/18804
- WO-A-93/21892
- DE-C1- 4 205 712
- DE-U1- 9 411 605
- JP-A- 5 146 484
- US-A- 3 895 741
- US-A- 4 284 209
- US-A- 4 880 409
- US-A- 5 101 984
- US-A1- 4 663 032
- US-A1- 5 874 208

## Description

### BACKGROUND OF THE INVENTION

This invention relates in general to medical laboratory devices, and in particular to a press for removing supernatant or fluids of different density from a flexible vessel after the vessel has been centrifuged.

Flexible vessels, similar to blood bags, can be used in biological suspension processing systems. Process requirements may require centrifugation of the vessel contents to separate cells or other relatively dense material from the carrier medium and subsequent removal of the supernatant. Centrifugation in the flexible vessel can be an attractive alternative to using traditional centrifugation tubes because the suspension can be left in the vessel. However, current techniques for transferring the suspension from a flexible vessel to centrifuge tubes introduce the possibility of contaminants enetering the suspension or exposing the laboratory operator to the suspension during the transfer process. This is highly dependent on operator skill and is labor, equipment and time intensive.

When traditional centrifuge tubes are utilized, supernatant is typically removed by manually pipetting the liquid out of the tube until the cell pellet/supernatant interface has been reached as determined by visual observation. This process works reasonably well but has the disadvantage of requiring careful implementation by a trained operator. Hand-controlled insertions of a pipette introduce the possibility of operator error and disturbance and resuspension of the cell pellet.

In some cases a density gradient fluid, such as Ficoll, is added to a starting fluid that is to be centrifuged, and upon centrifugation creates a defined band which separates layers of the starting fluid, and facilitates removal of a desired layer after centrifugation.

If a flexible centrifuge vessel is used, the supernatant can be pipetted, pumped or squeezed out of the vessel. The vessel's flexibility will cause it to compress or reduce in size as supernatant is removed. When the vessel compresses, cells in the cell pellet will move toward the exit opening, and some of the cells may be lost during removal of the supernatant.

The Baxter Plasma Extractor® is a commercially available press for squeezing plasma from a flexible blood bag. The blood bag is hung in the press between movable and stationary surfaces. The movable surface is released against the bag to squeeze out the liquid plasma. In normal operation, the press is allowed to close against a blood bag in which components have been separated. The press operator watches the material in the bag during the pressing operation. When the material to be retained in the bag visually moves up the bag to the exit, the operator closes a clamp on the exit tubing or grasps the movable press place handle to manually stop the flow of material. Although this process works well for the plasma extraction application, it may not be suitable for an application (such as processing cells for a therapy) where a maximum number of cells must be retained in the bag because there is a band of material where cells and supernatant are mixed together when the cell pellet is compressed and partially resuspended.

Presses for blood bags are also disclosed in the patent literature. For example, U.S. Patent No. 4,663,032 to Loos et al., issued May 5, 1987, discloses a press for driving plasma and buffy coat out of a centrifuged blood bag while leaving the red blood cells in the bag. The press includes a stationary plate and upper and lower movable plates. An elastic cushion is bonded between the upper plate and the piston that moves the plate. The press also includes a horizontal clamping element that extends between the upper and lower plates. The clamping element is extended against a cushion in the stationary plate to cut off the red blood cells from the buffy coat and plasma, in order to retain the red blood cells in the bag when it is compressed by the upper plate to drive out the buffy coat and plasma.

US-A1-4 663 032 discloses a press for driving a plasma layer and a buffy-coat layer in succession out of a centrifuged whole blood bag which has upper and lower press plates between which a clamping element is displaceable, all juxtaposed with a stationary plate so that after displacement to the lower press plate to drive off plasma and operation of the upper press plate to spread the buffy-coat layer in height, the clamping element can be actuated to clamp off the bag between the buffy-coat layer and the red cell concentrate so that advance of the upper press plate can drive off the buffy-coat layer.

WO 93/18804 A discloses a method for separating blood into blood components and an apparatus for removing blood components from a blood container which has at least one outlet opening. The method comprises introducing blood into a blood container which has at least one outlet opening in the upper limit of the container, centrifuging the blood container in a relatively high centrifugal field to form three mutually superimposed component layers, applying pressure to the blood container to remove the upper component through the outlet opening and, during or after this pressure stage, forming in the blood container a channel through which a second component is connected with the outlet opening or with a second outlet opening, and then applying further pressure to remove the second component through the channel formed. The apparatus for removing centrifuged and stratified blood components from a blood container having at least one outlet opening includes a housing which has mounted therein a first and a second pressure plate which are movable in relation to one another, a suspension means by means of which the blood container can be positioned between the pressure plates, and means for achieving relative movement of the pressure plates. The apparatus also includes a mandrel in the second pressure plate which is operative in forming a channel in the container as the blood container is compressed to remove the uppermost blood component, this channel connecting a blood container outlet opening with the bottom blood component and being formed only when the upper component has been removed from the blood container. US-A1-5 874 208 discloses a method and an extractor for pressing out plasma and buffy coat from a collapsible blood container, in which blood has been divided, by centrifugation, into a plasma layer, a buffy coat layer and a layer of red blood cells. The plasma and then the buffy coat are pressed out each through a separate outlet tube or through a common outlet tube, which is connected to the top portion of the blood container, a pulsating pressure being applied to a top section of the blood container during the end phase of the pressing-out of buffy coat. The extractor has a stationary support surface and a movable pressing member, between which the blood container is suspended and subjected to a compressive force in order to press out the plasma and then the buffy coat through the outlet tube. The extractor has one or more inflatable cushions arranged on the support surface or the pressing member or both on the same level as the top section of the blood container, and a device for pulsating the pressure in said cushions.

JP 05 146484A discloses a liquid separator which separates the blood components separated in layers in a blood bag and which prevents the blood component to be separated from remaining in the blood bag or from being mixed with other blood components.

DE 42 057 12 C2 discloses an auxiliary blood components separating device having two arms which can be moved toward one another and between which a blood bag can be arranged. The separating strips are arranged on the arms, the strips extending over the entire width of a blood bag and being arranged on the arms so as to point toward one another. In this way, the two components can be separated from one another and one of the two components can then be withdrawn from the blood bag.

### SUMMARY OF THE INVENTION

According to the present invention, a press having the features according to claim 1 and a method for removing a portion of biological material from a flexible process vessel having the features of claim 17 are provided. Preferred embodiments of the invention are defined in the respective dependent claims.

This invention relates to a press for removing supernatant from a flexible vessel, such as a flexible biological suspension process vessel.

The press for removing fluid from a flexible vessel according to the invention includes cooperating press members having an open position to insert the vessel between them, and a closed position to compress the vessel between them, at least one of the press members having a compliant pad on its press surface, the compliant pad compensating for variable thickness of the vessel when it is compressed, and the press being constructed to compress a first portion of the vessel less than a second portion of the vessel, and the press additionally including a barrier structure that creates a barrier that seals off the first portion of the vessel from the second portion of the vessel as the vessel is compressed; wherein the press in the closed position forms a space in a first portion of the press proximate to the first portion of the vessel so that the first portion of the vessel is compressed less than the second portion of the vessel, and wherein the compliant pad is located on the second portion of the press surface facing the second portion of the vessel, and not on the first portion of the press surface, and defines the space in the first portion of the press. The compliant pad compensates for variable thickness of the vessel when it is compressed.

According to an embodiment, either or both the press members can be made from a transparent material so the flow of material out of the vessel can be observed. As well, the compliant pad could be transparent. According to an alternative embodiment, a recessed area can be included in the plate that does not have the compliant material attached, thus creating a clearance pocket for the lower portion of the vessel so that portion would be uncompressed.

The barrier structure can be useful, for example, to prevent cells from migrating from the lower portion of the vessel into the upper portion of the vessel during the pressing operation, for controlling generally where cells migrate during the pressing operation, for preventing or controlling the loss of cells during the pressing operation, or to standardize or control the volume remaining in vessel.

The press can also include a vessel holder having a structure for adjusting the position of the vessel within the press. For instance, the vessel holder can be a vertically adjustable hanger or other posititioning device to allow for adjusting where the barrier structure makes contact with the vessel. The press can also include a mechanism for adjusting the location of the compliant pad and/or the barrier structure.

The present invention reduces the labor, equipment and time needed to separate a desired portion of a biological material and eliminates the possibility of introducing contaminants into the material or exposing a laboratory operator to the contents during a transfer process. As well, processing of biological materials using the present invention is less dependent on operator technique to achieve repeatable results.

Various advantages of this invention will become apparent to those skilled in the art from the following detailed description of the preferred embodiments, when read in light of the accompanying drawings.

### BRIEF DESCRIPTION OF THE DRAWINGS

Fig. 1 is a perspective view of a press for removing supernatant from a flexible vessel according to the invention.
Fig. 2 is a side view of the press of Fig. 1.
Fig. 3 is a top view of the press of Fig. 1.
Fig. 4 is a perspective view showing a flexible biological suspension processing vessel after centrifugation, and ready to be placed into a press according to the invention.
Fig. 5 is a perspective view showing the flexible vessel of Fig. 4 having been compressed in the press to remove the fluid or supernatant from the upper portion of the vessel.
Fig. 6 is an enlarged side view of the lower portion of the flexible vessel of Fig. 5 before the press is completely closed, showing that a barrier line is created between the second or upper portion of the vessel and the first or lower portion of the vessel.
Fig. 7 is an enlarged side view of the lower portion of the flexible vessel of Fig. 5 after the press is completely closed, showing that the supernatant has been removed from the upper portion of the vessel, and that the lower portion of the vessel containing the cell pellet remains substantially uncompressed.
Fig. 8 is an enlarged side view of the lower portion of the flexible vessel of Fig. 5 before the press is completely closed, showing two compliant strips creating two barrier lines that define first, second and third portions of the vessel.

### DETAILED DESCRIPTION OF THE INVENTION

The present invention relates to a press that has the capability of removing supernatant from a flexible vessel after a cell suspension has been centrifuged. The press features maximize the amount of cells retained in the bag without operator intervention.

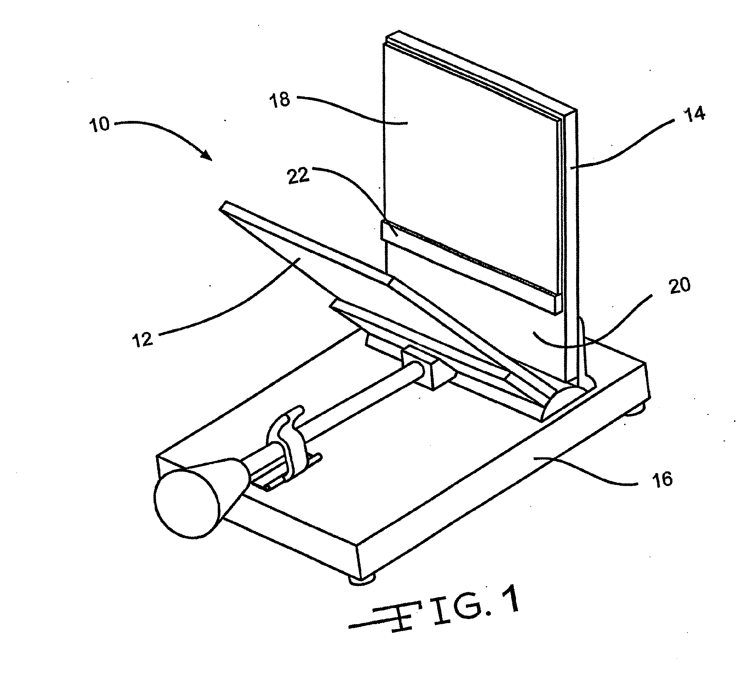

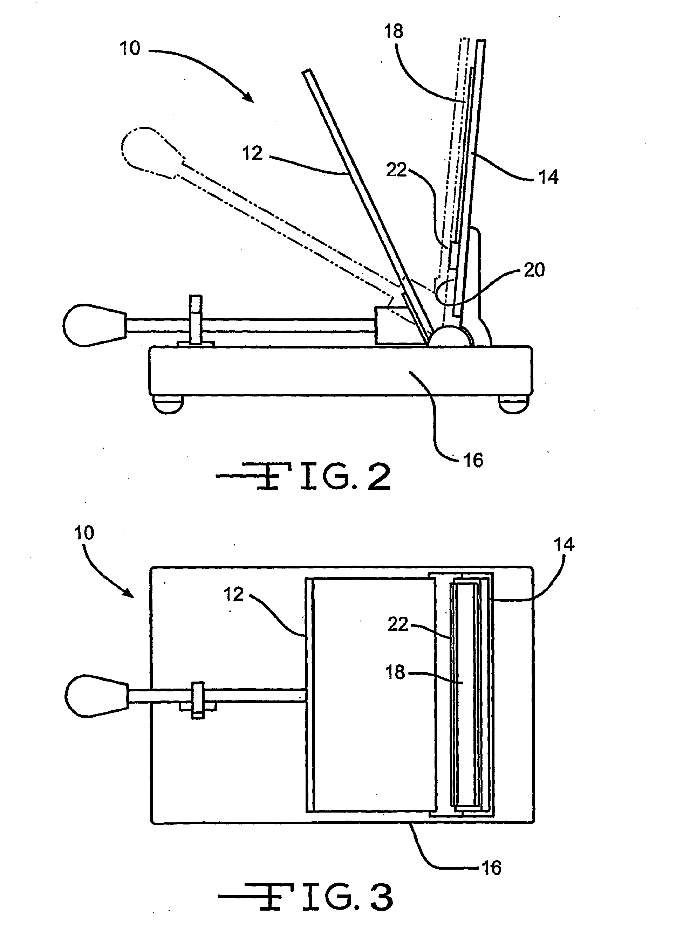

The press includes cooperating press members having an open position to insert the flexible vessel between them, and a closed position to compress the vessel between them. Any suitable configuration and number of press members can be used. In the embodiment shown in Figs. 1-3, the press 10 includes two press plates 12 and 14 that come together to press supernatant out of a flexible bag placed between them. Fig. 1 shows the press plates in the open position, and Fig. 2 shows the press plates in both the open position (solid lines) and the closed position (broken lines).

The press plates can both be movable, or one of the press plates can be movable while the other is stationary. In the illustrated embodiment, the second press plate 14 is mounted on the base 16 of the press in stationary position, and the first press plate 12 is pivotally mounted on the base. The plates could be hinged, tracked or otherwise movable, and may close from bottom to top as shown in the illustrated embodiment, from top to bottom, or from side to side. Any suitable structure can be used for the basic pressing mechanism. The movable plate(s) can be moved by spring operation or other means known in the art.

The press plates can be made from any suitable material, such as plastic, metal or wood. Preferably, at least one of the press plates is transparent to allow viewing of the vessel during the pressing operation. In the embodiment shown, the first press plate 12 is made from a transparent plastic material, although both of the press plates may be transparent. The press and the press plates are constructed and sized to hold and press supernatant from a flexible vessel, preferably a vessel for processing a biological suspension or fluid. The press can be used with different sizes and types of vessel, and the plates can be shaped to match design features of the vessel.

At least one of the press members has a compliant pad on its press surface. In the illustrated embodiment, the second press plate 14 has a layer of a compliant pad 18 on its press surface. The pad's compliance automatically compensates for the variable thickness in the vessel that typically occurs at the seams along the edges. This compliance allows the press to completely flatten the main body of the flexible vessel, causing essentially all of the liquid to be discharged. If the compliant pad is not used, the variation in vessel thickness holds the press slightly open, which prevents removal of some of the liquid. Any suitable material can be used for making the compliant pad. Preferably, the compliant pad is made from a foam material, such as a closed cell silicone foam. Many other compliant materials such as open cell or closed cell polyurethane, polyethylene and PVC could also be used. Materials other than foam such as soft rubber or a gas or fluid filled bladder can also serve as the compliant material.

The press is constructed to compress the lower portion of the vessel less than the upper portion. Any suitable structure can be used for this purpose. In a preferred embodiment, the press in the closed position forms a space in the lower portion of the press so that the lower portion of the vessel is compressed less than the upper portion. The space can be formed in any suitable manner. Preferably, as shown in Fig. 2, the space 20 is formed by locating the compliant pad 18 on the upper portion of the press surface and not on the lower portion. A space 20 equal to the pad thickness exists between the two press plates below the pad's bottom edge. Alternative press configurations can also be used so the lower portion of the vessel is uncompressed. Foam of adequate thickness could be attached to the upper portion of both press surfaces, creating an uncompressed space for the lower portion of the vessel. A recessed area can also be included in the plate that does not have the compliant material attached, thus creating a clearance pocket for the lower portion of the vessel so that portion would be uncompressed.

Reference to upper and lower portions of the vessel or press or components thereof, is only for convenience and understanding in view of the illustrated embodiment. The press plates may be designed to operate from top to bottom or side to side, and the vessel positioned for the desired operation. Thus, "lower" and "upper" might equally be referred to as "first" and "second".

The purpose of this construction is so that the upper portion of the vessel is compressed as much as possible while the lower portion of the vessel is left relatively uncompressed. Preferably, the press in the closed position substantially completely compresses the upper portion of the vessel while leaving the lower portion of the vessel substantially uncompressed.

In a biological suspension process vessel that has been centrifuged, a pellet of cells formes at the bottom and the supernatant, which may be unwanted, is above the cell pellet or other higher density biological material. Reference to a "cell pellet" herein is illustrative of a higher density biological material, and is not intended to limit the application. Further, the reference to a "supernatant" is illustrative of any fluid of lower density to be removed from the flexible vessel. The supernatant referred to herein is removed material, wanted or unwanted by the user. To remove the supernatant as completely as possible, the upper portion of the vessel can be pressed completely flat. The lower portion of the vessel where the cell pellet is located can be left uncompressed as much as possible so the pellet remains undisturbed by the pressing operation. The space allows the vessel portion below the pad to balloon out and remain uncompressed so the cell pellet remains undisturbed. The design distance from the bottom of the pad to the bottom of the vessel depends on the size of the cell pellet, the desired retained volume, and the vessel's material stiffness. The volume can be controlled by design of the compliant pad 18 and the compliant strip 22 to squeeze the vessel earlier or later during compression, or by choice of the compliant strip and pad profiles (rectangular, curved or other shapes). Stiffer vessel materials will move together earlier during the pressing operation than more compliant materials, so it may be appropriate for the bottom of the compliant pad to be positioned further from the bottom of the vessel than for a more flexible vessel. Similarly for a vessel containing a relatively large cell pellet, the bottom of the compliant pad may be positioned further from the bottom of the vessel than for a vessel containing a small cell pellet. As described below, the height of the vessel within the press can be adjusted to achieve a similar effect.

During the pressing operation, it is necessary to seal off the lower portion of the vessel, where the cell pellet is located, from the upper portion of the vessel to prevent cell migration up the vessel and loss out the exit opening during the pressing operation. Consequently, the press includes a barrier structure that creates a barrier between the lower portion of the vessel and the upper portion of the vessel when the vessel is compressed. This can be accomplished in any suitable manner. For example, the barrier structure can firmly squeeze the vessel between the upper and lower portions of the vessel. The barrier is created early in the pressing operation, before the majority of supernatant is pressed from the upper portion of the vessel.

Any suitable structure can be used for this purpose. In the illustrated embodiment, the barrier structure comprises a compliant strip 22 that is thicker than the compliant pad 18, and that is located along the bottom edge of the pad. Optionally, the compliant strip could be tapered or rounded. The compliant strip can have any suitable thickness, for example about twice the thickness of the pad. The barrier structure could be on one plate while the compliant pad is on the other plate. During the pressing operation, the compliant strip 22 squeezes firmly against the vessel to create a line of pressure against the vessel that forms a barrier before all of the supernatant is discharged from the upper portion of the vessel. The thicker compliant strip will be compressed more than the compliant pad when the press is fully closed to provide a higher force against the vessel at the barrier line than exists over the upper portion of the vessel where the supernatant is located. The resulting higher squeeze on the vessel at the barrier line seals the vessel between the cells and the supernatant and prevents cell migration up the vessel and loss of cells. The use of the compliant strip 22 thus helps minimize the loss of cells, maximize supernatant removal and maximize cell viability by not disturbing the desired materials.

Another suitable barrier structure could be a portion of the compliant pad that is stiffer than the remaining portion of the pad. Another barrier structure could be a strip portion of the compliant pad, along the lower edge of the pad, that is thicker than the remaining portion of the pad. The strip portion of the pad could have any suitable shape, such as a wedge shape or rounded shape. Another suitable structure could be an inflatable bladder that inflates from the bottom up, which could function as both the compliant pad and the barrier structure.

In the illustrated embodiment, the bottom ends of the press plates 12 and 14 are mounted close to each other, so that during the pressing operation the barrier structure squeezes the vessel to form a barrier between the bottom of the vessel and the top before the top of the vessel is compressed. Any other suitable structure of the press plates could be used to achieve this.

Centrifuged suspensions frequently have different cell concentrations. Consequently, similar suspension volumes yield different sized cell pellets. A variant that could be added to the press is a structure for adjusting the height of the vessel within the press (not shown). For example, the height adjustment structure could be a vertically adjustable hanger or other positioning device at the top of the press. The vessel height could be adjusted so a specific distance is achieved between the top edge of the cell pellet and the bottom edge of the compliant pad. This adjustability will help minimize the amount of supernatant left in the bottom portion of the vessel below the barrier line and maximize the amount of supernatant removed from the vessel. The press can also include a mechanism for adjusting the location of the compliant pad and/or the location of the barrier structure, to adjust for the vessel size or the pellet size. The press can further include a mechanism other than a hanger for adjusting the location of the vessel. By way of example and not limitation, alternative positioning devices (not shown) may include devices such as clips or clamps or straps; releasable adhesive surfaces; or Velcro surfaces, interlocking profiles or zippered closures on the vessel that combine with one of a plurality of mating profiles or zipper elements on an opposing surface and other adjustable positioning devices.

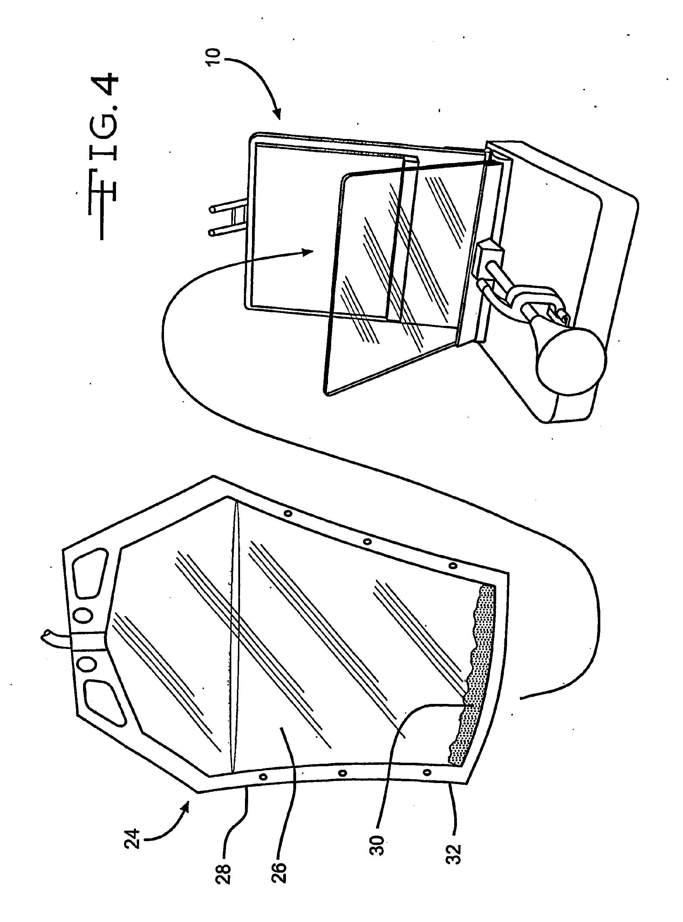

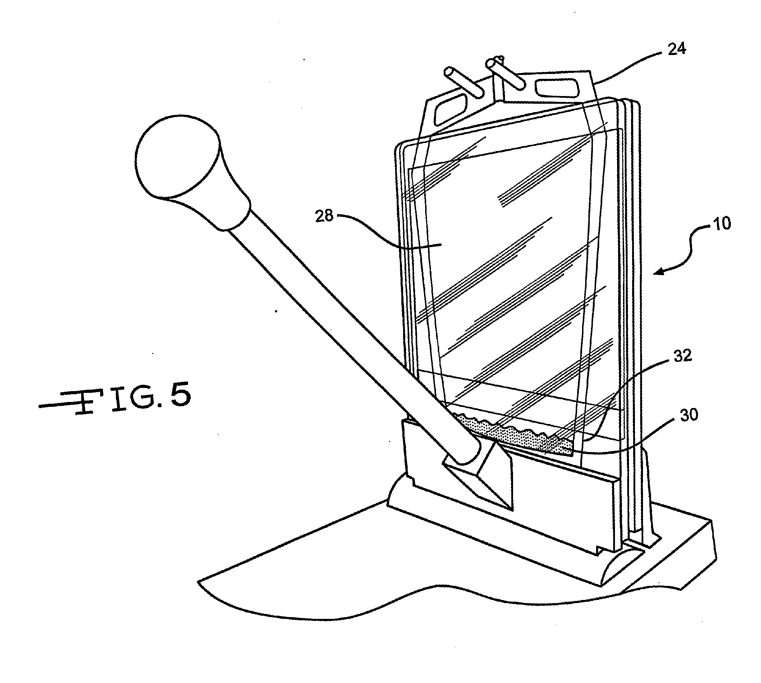

Figs. 4-7 illustrate a pressing operation using the press of the invention. In Fig. 4, a flexible biological suspension processing vessel 24 is shown after centrifugation. The vessel 24 contains supernatant 26 in the upper portion 28 of the vessel and a cell pellet 30 in the lower portion 32 of the vessel. The vessel is ready to be placed into an open press 10. In Fig. 5, the vessel 24 has been compressed in the press 10 to remove the supernatant from the upper portion 28 of the vessel. The lower portion 32 of the vessel containing the cell pellet 30, which is located in the space 20, is substantially uncompressed.

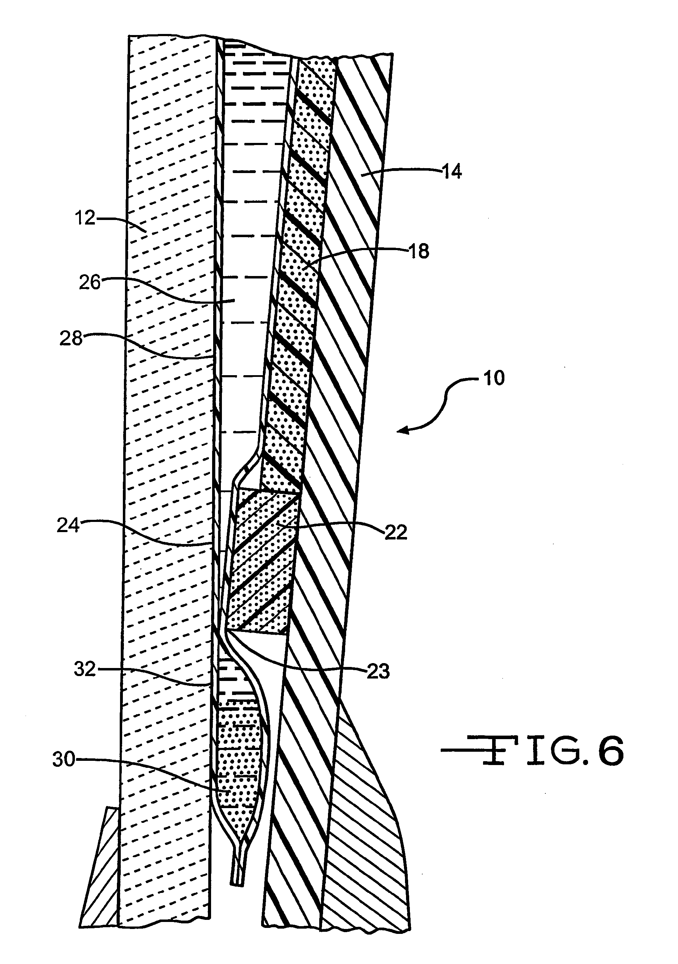

Fig. 6 shows the press just before it is completely closed. It is seen that the compliant strip 22 contacts the vessel 24 to create a barrier line 23 between the first or lower portion 32 of the vessel and the second or upper portion 28 of the vessel, before the supernatant 26 has been pressed out of the upper portion of the vessel. This ensures that the cell pellet 30 in the lower portion of the vessel does not leave with the supernatant.

It may be understood by reference to Fig. 6 that the compliant strip 22 could be shaped so as not to create a barrier line until a certain level of compression of the vessel is reached. As well, it may be designed to create a barrier line at the outset, as shown, at the end of the pressing step, or at some intermediate time during the pressing step.

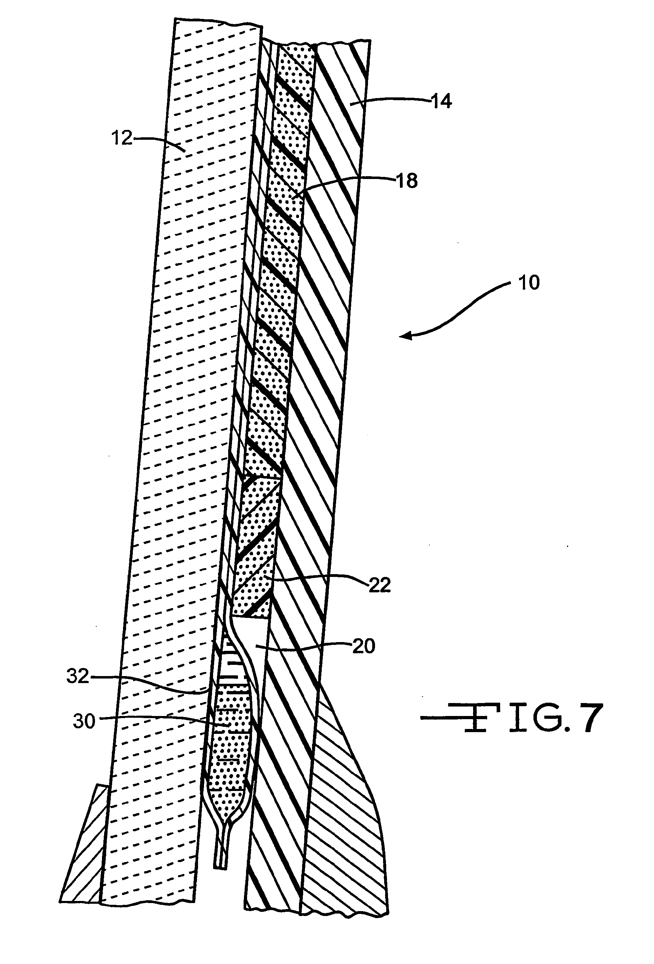

Fig. 7 shows the press in a closed position with the supernatant removed. The lower portion 32 of the vessel is ballooned out in the space 20 and remains uncompressed so that the cell pellet 30 remains undisturbed.

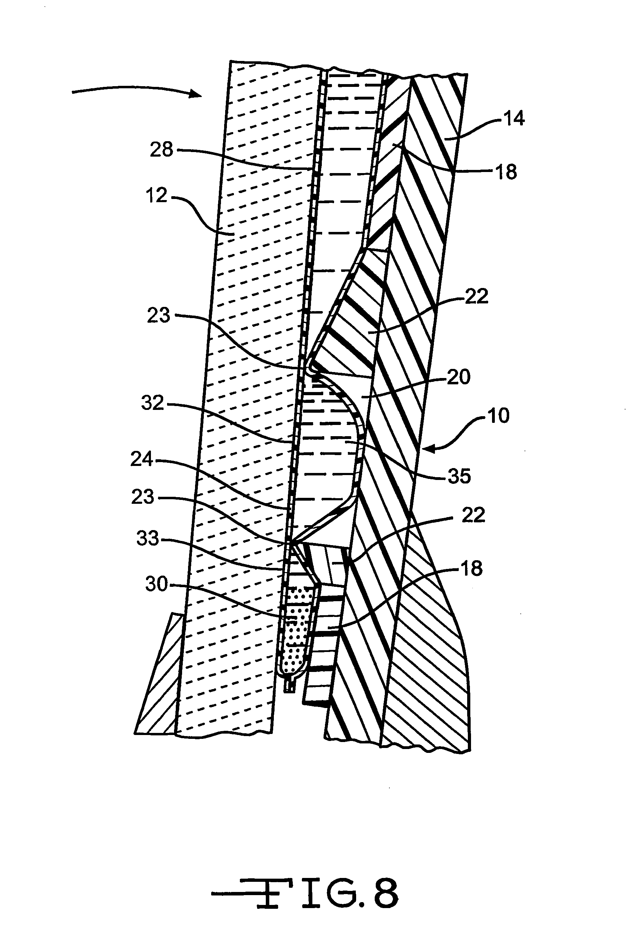

As well, different density gradients of biological materials, not necessarily positioned at the bottom of the vessel, may be of interest. In a further embodiment of the present invention shown in Fig. 8, two compliant strips 22 may be provided to define the first portion 32 between the second portion 28 and the third portion 33 and to isolate an intermediate density gradient 3 5 containing biological material in the first portion. Supernatant and higher density material that is not desired to remain in the vessel can be exhausted from above and below the intermediate density gradient 35 through exhaust ports on the ends or lateral exhaust ports on the sides of the vessel (not shown). Where fluid is to be pressed through an exhaust port the compliant pad 18 can be countoured to so direct the fluid as the press is closing. For example, where fluid is exhausted through a lateral exhaust port, the compliant pad 18 can be wedge shaped from side to side or otherwise contoured to encourage the liquid movement toward the lateral exhaust port. As may be understood, several portions of the vessel could be isolated in this way where more than one density gradient of the biological material is of interest. The compliant strips 22 may be of different heights and profiles (by way of example and not limitation, flat, pointed, rounded, wedge-shaped) as needed to compress the vessel and isolate the density gradient of interest. In this embodiment, adjustment of the vessel position or adjustment of the compliant strip positions may also help maximize the percentage capture of cells or other biological material of interest and minimize the volume in which they are captured. This approach to separation of biological material also renders the process less dependent on operator technique.

The press could be automated to achieve maximum efficiency of operation. The automated press could include sensors to detect conditions related to the pressing operation such as volume or location of the cell pellet or other biological material of interest, degree of compression, and completion of supernatant removal. The use of a transparent material for at least one of the press plates makes it possible for a sensor to detect the progress of the pressing operation. The sensor could cause an adjustment in the vessel hanging device or the compliant pad positioning device. A pressure sensor, a limit switch or a position switch could be used to determine when the pressing is complete. An optical sensor could be used to determine when all the supernatant has been removed from the upper portion of the vessel. The press could be rotated to allow supernatant to drain.

The present invention also relates to a method of using a closed process for removing supernatant from a flexible process vessel according to claim 17.

Any suitable components can be used in the closed system in addition to the flexible vessel and the container, as long the components are sterilized and as long as they keep the system closed. For example, the components can include sterile connecting devices (SCD) such as SCD tubing and a SCD tubing welder to make connections asceptically between the vessel and the container. The vessel and the container can each have connections that are compatible with the SCD tubing.

Batch biological laboratory processes are often performed in a sterile biological safety cabinet to protect product sterility as various fluids, necessary to the process, are utilized and to protect the operator if the product is hazardous. The closed process of the invention allows biological processes to be performed in a non-sterile laboratory environment, thereby saving time and money.

In accordance with the provisions of the patent statutes, the principle and mode of operation of this invention have been explained and illustrated in its preferred embodiments. However, it must be understood that this invention may be practiced otherwise than as specifically explained and illustrated without departing from its scope as defined in the appended claims.

## Claims

1. A press (10) for removing fluid from a flexible vessel, the press (10) including cooperating press members having an open position to insert the vessel between them, and a closed position to compress the vessel between them, at least one of the press members having a compliant pad (18) on its press surface, the compliant pad (18) compensating for variable thickness of the vessel when it is compressed, and the press (10) being constructed to compress a first portion (32) of the vessel less than a second portion (28) of the vessel, and
the press additionally including a barrier structure that creates a barrier that seals off the first portion (32) of the vessel from the second portion (28) of the vessel as the vessel is compressed;
wherein the press (10) in the closed position forms a space (20) in a first portion of the press (10) proximate to the first portion (32) of the vessel so that the first portion (32) of the vessel is compressed less than the second portion (28) of the vessel, and
wherein the compliant pad (18) is located on the second portion of the press surface, and not on the first portion of the press surface, and defines the space (20) in the first portion (32) of the press (10);
**characterized in that**
the compliant pad faces the second portion (28) of the vessel.

2. A press (10) according to claim 1 wherein the barrier structure creates the barrier when the barrier structure is at least partially under compression.

3. A press (10) according to claim 1 wherein the barrier structure comprises a compliant strip (22) that is thicker than the compliant pad (18).

4. A press (10) according to claim 1 wherein the barrier structure comprises a portion of the compliant pad (18) that is stiffer than the remaining portion of the pad (18).

5. A press (10) according to claim 1 wherein the compliant pad (18) comprises a foam pad.

6. A press (10) according to claim 3 wherein the compliant strip (22) comprises a foam strip.

7. A press (10) according to claim 1 wherein the press (10) in the closed position substantially completely compresses the second portion (28) of the vessel while leaving the first portion (32) of the vessel substantially uncompressed.

8. A press (10) according to claim 1 wherein the press members comprise a pair of press plates (12, 14).

9. A press (10) according to claim 8 wherein at least a portion of one or more of the press plates (12, 14) is transparent to allow viewing of the vessel during the pressing operation.

10. A press (10) according to claim 8 wherein the press plates (12, 14) include opposed first ends proximate to the first portion (32) of the vessel, and opposed second ends proximate to the second portion (28) of the vessel, and the press plates (12, 14) are mounted such that opening and closing the press (10) causes more relative movement between the second ends than between the first ends.

11. A press (10) according to claim 1 further comprising two barrier structures that each create a barrier line, and wherein the compliant pad (18) defines a substantially uncompressed portion between the barrier lines, and at least one substantially compressed portion outside the portion between the barrier lines, when the press (10) is in a closed position.

12. A press (10) according to claim 8 wherein the press (10) additionally ineludes a base (16), and wherein one of the press plates (12, 14) is mounted in stationary position on the base (16) and the other of the press plates (12, 14) is pivotally mounted on the base (16).

13. A press (10) according to claim 1 wherein the press (10) additionally includes a mechanism for adjusting the location of at least one of the vessel, the barrier structure, and the compliant pad (18).

14. A press (10) according to claim 1 wherein the press includes a vessel holder having a structure for adjusting the height of the vessel within the press (10).

15. A press (10) according to claim 14 wherein the vessel holder comprises a vertically adjustable hanger.

16. A press according to claim 1 wherein the barrier structure is included as part of at least one of the press members.

17. A method of removing a portion of biological material from a flexible process vessel comprising the steps of:
(1) providing a flexible process vessel containing fluid biological material at least partially separated by density;
(2) placing the vessel into a press (10) for removing the material of a selected density gradient from the vessel, the press (10) including cooperating press members having an open position to insert the vessel between them, and a closed position to compress the vessel between them, the press (10) being constructed to compress a first portion (32) of the vessel less than a second portion (28) of the vessel, at least one of the cooperating press members including a compliant pad (18) on its press surface, and at least one of the press members further including a barrier structure constructed to define a barrier that seals off the first portion (32) of the vessel from the second portion (28) of the vessel as the vessel is compressed;
and
(3) pressing the vessel to remove the material of a selected density gradient from the vessel while retaining the remaining biological material in the vessel;
wherein the method further includes the steps of adjusting the position of at least one of the vessel, the compliant pad (18) and the barrier structure prior to the step of pressing to define the first and second portions (32, 28),
wherein the press (10) in the closed position forms a space (20) in a first portion of the press (10) proximate to the first portion (32) of the vessel so that the first portion (32) of the vessel is compressed less than the second portion (28) of the vessel, and
wherein the compliant pad (18) is located on the second portion of the press surface, and not on the first portion of the press surface, and defines the space (20) in the first portion (32) of the press (10);
**characterized in that**
the compliant pad faces the second portion (28) of the vessel.

18. The method of claim 17 wherein the compliant pad (18) on its press surface is constructed to compress a first portion (32) of the vessel less than second and third portions (28, 33) of the vessel, wherein the barrier structure is a first barrier structure, and further comprising a second barrier structure disposed on one of the press members, the two barrier structures defining the first portion (32) of the vessel between the second and third portions (28, 33) of the vessel;
wherein the method further includes the step of pressing the vessel to remove the material of a selected density gradient from the second and third portions (28, 33) of the vessel while retaining the remaining biological material in the first portion (32) of the vessel.

19. The method of claim 17 wherein at least a portion of one or more of the press plates (12, 14) is transparent, and the step of pressing includes the step of monitoring the vessel through a transparent portion of a press plate (12, 14) during the pressing operation.

## Patentansprüche

1. Presse (10) zur Entfernung von Fluid aus einem flexiblen Behälter, wobei die Presse (10) umfasst: zusammenwirkende Pressenelemente mit einer offenen Position, um den Behälter zwischen ihnen einzusetzen, und einer geschlossenen Position, um den Behälter zwischen ihnen zusammenzudrücken, wobei wenigstens eines der Pressenelemente ein nachgiebiges Polster (18) auf seiner Pressenoberfläche hat, wobei das nachgiebige Polster (18) die variable Dicke des Behälters ausgleicht, wenn er zusammengedrückt wird, und die Presse (10) aufgebaut ist, um einen ersten Abschnitt (32) des Behälters weniger als einen zweiten Abschnitt (28) des Behälters zusammenzudrücken, und
die Presse zusätzlich eine Barrierenstruktur hat, die eine Barriere erzeugt, die den ersten Abschnitt (32) des Behälters (32) gegen den zweiten Abschnitt (28) des Behälters abdichtet, wenn der Behälter zusammengedrückt wird;
wobei die Presse (10) in der geschlossenen Position in einem ersten Abschnitt der Presse (10) nahe dem ersten Abschnitt (32) des Behälters einen Raum (20) bildet, so dass der erste Abschnitt (32) des Behälters weniger als der zweite Abschnitt (28) des Behälters zusammengedrückt wird, und
wobei das nachgiebige Polster (18) auf dem zweiten Abschnitt der Pressenoberfläche und nicht auf dem ersten Abschnitt der Pressenoberfläche angeordnet ist und den Raum (20) in dem ersten Abschnitt (32) der Presse (10) definiert;
**dadurch gekennzeichnet, dass**
das nachgiebige Polster dem zweiten Abschnitt (28) des Behälters zugewandt ist.

2. Presse (10) nach Anspruch 1, wobei die Barrierenstruktur die Barriere erzeugt, wenn die Barrierenstruktur wenigstens teilweise unter Druck steht.

3. Presse (10) nach Anspruch 1, wobei die Barrierenstruktur einen nachgiebigen Streifen (22) umfasst, der dicker als das nachgiebige Polster (18) ist.

4. Presse (10) nach Anspruch 1, wobei die Barrierenstruktur einen Abschnitt des nachgiebigen Polsters (18) umfasst, der steifer als der restliche Abschnitt des Polsters (18) ist.

5. Presse (10) nach Anspruch 1, wobei das nachgiebige Polster (18) ein Schaumpolster umfasst.

6. Presse nach Anspruch 3, wobei der nachgiebige Streifen (22) einen Schaumstreifen umfasst.

7. Presse (10) nach Anspruch 1, wobei die Presse (10) in der geschlossenen Position den zweiten Abschnitt (28) des Behälters im Wesentlichen vollständig zusammendrückt, während sie den ersten Abschnitt (32) des Behälters im Wesentlichen unzusammengedrückt lässt.

8. Presse (10) nach Anspruch 1, wobei die Pressenelemente ein Paar von Pressenplatten (12, 14) umfassen.

9. Presse (10) nach Anspruch 8, wobei wenigstens ein Abschnitt einer oder mehrerer der Pressenplatten (12, 14) transparent ist, um das Betrachten des Behälters während des Pressarbeitsgangs zuzulassen.

10. Presse (10) nach Anspruch 8, wobei die Pressenplatten (12, 14) entgegengesetzte erste Enden nahe dem ersten Abschnitt (32) des Behälters und entgegengesetzte zweite Enden nahe dem zweiten Abschnitt (28) des Behälters umfassen, und die Pressenplatten (12, 14) derart montiert sind, dass das Öffnen und Schließen der Presse (10) mehr Relativbewegung zwischen den zweiten Enden als zwischen den ersten Enden bewirkt.

11. Presse (10) nach Anspruch 1, die ferner zwei Barrierenstrukturen umfasst, die jeweils eine Sperrlinie erzeugen, und wobei das nachgiebige Polster (18) einen im Wesentlichen nicht zusammengedrückten Abschnitt zwischen den Sperrlinien und wenigstens einen im Wesentlichen zusammengedrückten Abschnitt außerhalb des Abschnitts zwischen den Sperrlinien definiert, wenn die Presse (10) in einer geschlossenen Position ist.

12. Presse (10) nach Anspruch 8, wobei die Presse (10) zusätzlich eine Basis (16) umfasst, und wobei eine der Pressenplatten (12, 14) in einer stationären bzw. ortsfesten Position auf der Basis (16) montiert ist und die andere der Pressenplatten (12, 14) dreh- bzw. schwenkbar auf der Basis (16) montiert ist.

13. Presse (10) nach Anspruch 1, wobei die Presse (10) zusätzlich einen Mechanismus zum Einstellen der Lage des Behälters und/oder der Barrierenstruktur und/oder des nachgiebigen Polsters (18) umfasst.

14. Presse (10) nach Anspruch 1, wobei die Presse eine Behälterhalterung mit einer Struktur zum Einstellen der Höhe des Behälters innerhalb der Presse (10) umfasst.

15. Presse (10) nach Anspruch 14, wobei die Behälterhalterung einen vertikal einstellbaren Aufhänger umfasst.

16. Presse nach Anspruch 1, wobei die Barrierenstruktur als Teil wenigstens eines der Pressenelemente enthalten ist.

17. Verfahren zur Entfernung eines Teils von biologischem Material aus einem flexiblen Verfahrensbehälter, das die folgenden Schritte umfasst:
(1) Bereitstellen eines flexiblen Verfahrensbehälters, der fluides biologisches Material enthält, das zumindest teilweise nach der Dichte getrennt ist;
(2) Anordnen des Behälters in einer Presse (10) zur Entfernung des Materials mit einem ausgewählten Dichtegradienten aus dem Behälter, wobei die Presse (10) zusammenwirkende Pressenelemente mit einer offenen Position, um den Behälter zwischen ihnen einzusetzen, und einer geschlossenen Position, um den Behälter zwischen ihnen zusammenzudrücken, umfasst, wobei die Presse (10) derart aufgebaut ist, dass sie einen ersten Abschnitt (32) des Behälters weniger als einen zweiten Abschnitt (28) des Behälters zusammendrückt, wobei wenigstens eines der zusammenwirkenden Pressenelemente ein nachgiebiges Polster (18) auf seiner Pressenoberfläche enthält, und wenigstens eines der Pressenelemente ferner eine Barrierenstruktur enthält, die aufgebaut ist, um eine Barriere zu definieren, die den ersten Abschnitt (32) des Behälters (32) gegen den zweiten Abschnitt (28) des Behälters abdichtet, wenn der Behälter zusammengedrückt wird;
und
(3) Zusammendrücken des Behälters, um das Material mit einem ausgewählten Dichtegradienten aus dem Behälter zu entfernen, während das restliche biologische Material in dem Behälter zurückgehalten wird;
wobei das Verfahren ferner vor dem Schritt des Zusammendrückens die Schritte des Einstellens der Position des Behälters und/oder des nachgiebigen Polsters (18) und/oder der Barrierenstruktur umfasst, um die ersten und zweiten Abschnitte (32, 34) zu definieren,
wobei die Presse (10) in der geschlossenen Position in einem ersten Abschnitt der Presse (10) nahe dem ersten Abschnitt (32) des Behälters einen Raum (20) bildet, so dass der erste Abschnitt (32) des Behälters weniger als der zweite Abschnitt (28) des Behälters zusammengedrückt wird, und
wobei das nachgiebige Polster (18) auf dem zweiten Abschnitt der Pressenoberfläche und nicht auf dem ersten Abschnitt der Pressenoberfläche angeordnet ist und den Raum (20) in dem ersten Abschnitt (32) der Presse (10) definiert;
**dadurch gekennzeichnet, dass**
das nachgiebige Polster dem zweiten Abschnitt (28) des Behälters zugewandt ist.

18. Verfahren nach Anspruch 17, wobei das nachgiebige Polster (18) auf seiner Pressenoberfläche aufgebaut ist, um einen ersten Abschnitt (32) des Behälters weniger als zweite und dritte Abschnitte (28, 33) des Behälters zusammenzudrücken, wobei die Barrierenstruktur eine erste Barrierenstruktur ist, und ferner eine zweite Barrierenstruktur umfasst, die auf einem der Pressenelemente angeordnet ist, wobei die zwei Barrierenstrukturen den ersten Abschnitt (32) des Behälters zwischen den zweiten und dritten Abschnitten (28, 33) des Behälters definieren;
wobei das Verfahren ferner den Schritt des Zusammendrückens des Behälters umfasst, um das Material mit einem ausgewählten Dichtegradienten aus den zweiten und dritten Abschnitten (28, 33) des Behälters zu entfernen, während das restliche biologische Material in dem ersten Abschnitt (32) des Behälters zurückgehalten wird.

19. Verfahren nach Anspruch 17, wobei wenigstens ein Abschnitt einer oder mehrerer Pressenplatten (12, 14) transparent ist und der Schritt des Zusammendrückens den Schritt des Überwachens des Behälters durch einen transparenten Abschnitt einer Pressenplatte (12, 14) während des Zusammendrückarbeitsgangs umfasst.

## Revendications

1. Presse (10) destinée à enlever un fluide depuis un contenant souple, la presse (10) incluant des éléments de pressage en coopération qui ont une position ouverte pour insérer le contenant entre eux et une position fermée pour comprimer le contenant entre eux, au moins un des éléments de pressage ayant un coussinet de support souple (18) sur sa surface de pressage, le coussinet de support souple (18) compensant une épaisseur variable du contenant lorsqu'il est comprimé, et la presse (10) étant construite pour moins comprimer une première partie (32) du contenant qu'une seconde partie (28) du contenant, et
la presse inclut en supplément une structure de barrière qui crée une barrière qui étanche la première partie (32) du contenant envers la seconde partie (28) du contenant lorsque le contenant est comprimé ;
**caractérisée en ce que** le coussinet de support souple fait face à la seconde partie (28) du contenant,
dans laquelle la presse (10) dans la position fermée forme un espace (20) dans une première partie de la presse (10) à proximité de la première partie (32) du contenant de telle sorte que la première partie (32) du contenant est moins comprimée que la seconde partie (28) du contenant, et
dans laquelle le coussinet de support souple (18) est placé sur la seconde partie de la surface de pressage et non sur la première partie de la surface de pressage et définit l'espace (20) dans la première partie (32) de la presse (10).

2. Presse (10) selon la revendication 1, dans laquelle la structure de barrière crée la barrière lorsque la structure de barrière est sous compression au moins en partie.

3. Presse (10) selon la revendication 1, dans laquelle la structure de barrière comprend une bande de support souple (22) qui est plus épaisse que le coussinet de support souple (18).

4. Presse (10) selon la revendication 1, dans laquelle la structure de barrière comprend une partie du coussinet de support souple (18) qui est plus rigide que la partie restante du coussinet (18).

5. Presse (10) selon la revendication 1, dans laquelle le coussinet de support souple (18) comprend un coussinet de mousse.

6. Presse (10) selon la revendication 3, dans laquelle la bande de support souple (22) comprend une bande de mousse.

7. Presse (10) selon la revendication 1, dans laquelle la presse (10) dans la position fermée comprime la seconde partie (28) du contenant sensiblement au complet tout en laissant la première partie (32) du contenant sensiblement non comprimée.

8. Presse (10) selon la revendication 1, dans laquelle les éléments de pressage comprennent une paire de plaques de pressage (12, 14).

9. Presse (10) selon la revendication 8, dans laquelle au moins une partie d'une ou des plusieurs plaques de pressage (12, 14) est transparente afin de permettre la visualisation du contenant pendant l'opération de pressage.

10. Presse (10) selon la revendication 8, dans laquelle les plaques de pressage (12, 14) incluent des premières extrémités opposées à proximité de la première partie (32) du contenant et opposées aux secondes extrémités à proximité de la seconde partie (28) du contenant, et dans la quelle les plaques de pressage (12, 14) sont montées de telle sorte que l'ouverture et la fermeture de la presse (10) provoquent un mouvement relatif plus grand entre les secondes extrémités qu'entre les premières extrémités.

11. Presse (10) selon la revendication 1, comprenant en outre deux structures de barrière qui créent chacune une ligne de barrière et dans laquelle le coussinet de support souple (18) définit une partie sensiblement non comprimée entre les lignes de barrière et au moins une partie sensiblement comprimée en dehors de la partie entre les lignes de barrière lorsque la presse (10) est dans une position fermée.

12. Presse (10) selon la revendication 8, dans laquelle la presse (10) inclut en supplément une base (16) et dans laquelle une parmi les plaques de pressage (12, 14) est montée dans une position stationnaire sur la base (16) et l'autre des plaques de pressage (12, 14) est montée avec faculté de pivotement sur la base (16).

13. Presse (10) selon la revendication 1, dans laquelle la presse (10) inclut en supplément un mécanisme pour ajuster l'emplacement d'au moins un composant parmi le contenant, la structure de barrière et le coussinet de support souple (18).

14. Presse (10) selon la revendication 1, dans laquelle la presse inclut un support de contenant qui a une structure pour ajuster la hauteur du contenant à l'intérieur de la presse (10).

15. Presse (10) selon la revendication 14, dans laquelle le support de contenant comprend un palier suspendu ajustable à la verticale.

16. Presse selon la revendication 1, dans laquelle la structure de barrière est incluse comme une partie d'au moins un des éléments de pressage.

17. Procédé destiné à retirer une partie d'un matériau biologique depuis un contenant de traitement souple, comprenant les étapes suivantes :
(1) la fourniture d'un contenant de traitement souple qui contient du matériau biologique fluidique séparé au moins en partie par la densité ;
(2) la mise en place du contenant dans une presse (10) afin de retirer le matériau d'un gradient de densité sélectionné depuis le contenant, la presse (10) incluant des éléments de pressage en coopération qui ont une position ouverte pour insérer le contenant entre eux et une position fermée pour comprimer le contenant entre eux, la presse (10) étant construite pour moins comprimer une première partie (32) du contenant qu'une seconde partie (28) du contenant, au moins un des éléments de pressage en coopération incluant un coussinet de support souple (18) sur sa surface de pressage, et au moins un des éléments de pressage incluant en outre une structure de barrière construite pour définir une barrière qui étanche la première partie (32) du contenant envers la seconde partie (28) du contenant lorsque le contenant est comprimé ;
et
(3) le pressage du contenant afin de retirer le matériau d'un gradient de densité sélectionné depuis le contenant tout en retenant le matériau biologique restant dans le contenant ;
dans lequel le procédé inclut en outre les étapes consistant à ajuster la position d'au moins un composant parmi le contenant, le coussinet de support souple (18) et la structure de barrière avant d'exécuter l'étape de pressage afin de définir la première partie et la seconde partie (32, 28),
**caractérisé en ce que** le coussinet de support souple fait face à la seconde partie (28) du contenant,
dans lequel la presse (10) dans la position fermée forme un espace (20) dans une première partie de la presse (10) à proximité de la première partie (32) du contenant de telle sorte que la première partie (32) du contenant est moins comprimée que la seconde partie (28) du contenant, et
dans lequel le coussinet de support souple (18) est placé sur la seconde partie de la surface de pressage et non sur la première partie de la surface de pressage et définit l'espace (20) dans la première partie (32) de la presse (10).

18. Procédé selon la revendication 17, dans lequel le coussinet de support souple (18) sur sa surface de pressage est construit pour moins comprimer une première partie (32) du contenant qu'une seconde partie et qu'une troisième partie (28, 33) du contenant, dans lequel la structure de barrière est une première structure de barrière, et comprenant en outre une seconde structure de barrière placé sur un composant parmi les éléments de pressage, les deux structures de barrière qui définissent la première partie (32) du contenant entre la seconde partie et la troisième partie (28, 33) du contenant ;
dans lequel le procédé inclut en outre l'étape consistant à presser le contenant afin de retirer le matériau d'un gradient de densité sélectionné depuis la seconde partie et la troisième partie (28, 33) du contenant tout en retenant le matériau biologique restant dans le contenant.

19. Procédé selon la revendication 17, dans lequel au moins une partie d'une ou des plusieurs plaques de pressage (12, 14) est transparente et dans lequel l'étape de pressage inclut l'étape de monitorage du contenant par l'intermédiaire d'une partie transparente d'une plaque de pressage (12, 14) pendant l'opération de pressage.
